# EUROPEAN PATENT APPLICATION

(11) **EP 1 992 617 A1**
(43) Date of publication of application: **19.11.2008**
(21) Application number: 07009706.8
(22) Date of filing: 15.05.2007
(51) Int. Cl.: C07D 231/14, A01N 43/56

(54) **Optically active ethyl amides**

(71) Applicant: Syngenta Participations AG, 4058 Basel (CH)
(72) Inventor: The designation of the inventor has not yet been filed

(57) **Abstract**

Compounds of the formula I 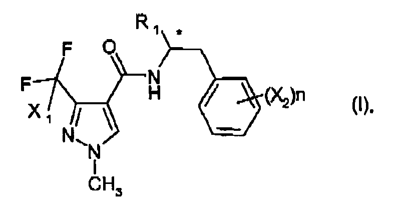 wherein R₁ is C₁-C₃alkyl, CF₃ or CF₂H; X₁ is hydrogen or fluoro; n is 2 or 3; each X₂ independently of each other stands for chloro, bromo, fluoro, CH₃ or CF₃; having an optical activity [α]_{D} of higher than 0° when dissolved in an achiral solvent; are suitable for use as microbiocides.

## Description

The present invention relates to novel optically active ethyl amides having microbiocidal, in particular fungicidal, activity. It further relates to intermediates used in the preparation of these compounds, to compositions which comprise these compounds and to their use in agriculture or horticulture for controlling or preventing infestation of plants by phytopathogenic microorganisms, preferably fungi.

N-[2-(pyridinyl)ethyl]-carboxamide derivatives and their use as fungicides are described in WO 04/74280, WO 05/85238, WO 06/8193 and WO 06/8194. Pyrazole-4-carboxylic acid amide derivatives and their use as pest-controlling agents are described in JP-2001-342179. Similar compounds are also known in other fields of technology, for example, the use of pyrazole-amides and sulfonamides as pain therapeutics is described in WO 03/37274.

It has been found that novel optically active ethyl amides have microbiocidal activity.

The present invention thus provides a compound of the formula I 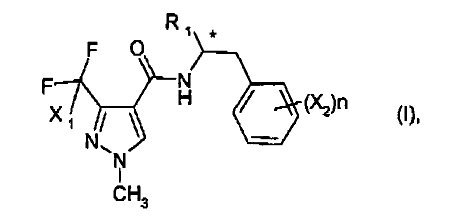 wherein R₁ is C₁-C₃alkyl, CF₃ or CF₂H; X₁ is hydrogen or fluoro; n is 2 or 3; each X₂ independently of each other stands for chloro, bromo, fluoro, CH₃ or CF₃; having an optical activity [α]_{D} of higher than 0° when dissolved in an achiral solvent.

The alkyl groups occurring in the definitions of the substituents can be straight-chain or branched and are, for example, methyl, ethyl, *n*-propyl or *iso*-propyl.

The compounds of formula I have one chiral carbon atom, which is highlighted in the depicted structure above by an asterisk.

Compounds of formula I can occur as enantiomeric pure (+)-enantiomers (enantiomeric excess (ee) > 99%) or as mixtures of the (+)- and (-)-enantiomers having an enantiomeric excess of the (+)-enantiomer.

Both enantiomers can be clearly distinguished by their optical activity [α]_{D} when dissolved in an achiral solvent: one has an optical activity [α]_{D} higher than 0° (the (+)-compound according to the invention) and one has an optical activity [α]_{D} lower than 0° (the (-)-compound according to the invention).

It has been found out that the (+)-compounds have higher microbiocidal activity as the (-)-compounds or as the racemic mixtures of both compounds.

The invention preferably provides compounds of the formula 1, wherein X₁ is hydrogen.

The invention preferably provides compounds of the formula 1, wherein at least one substituent X₂ is located in the ortho-position at the phenyl ring.

The invention preferably provides compounds of the formula I, wherein each X₂ is chloro. In one embodiment of the invention n is 2. In another embodiment of the invention n is 3.

In one embodiment of the invention R₁ is methyl. In another embodiment of the invention R₁ is ethyl. In another embodiment of the invention R₁ is CF₃.

The invention preferably provides compounds of the formula I with an enantiomeric excess of the (+)-enantiomer of at least 50%.

The invention preferably provides compound of the formula I with an enantiomeric excess of the (+)-enantiomer of at least 75%.

In one embodiment of the invention, the compound of the formula I is an enantiomeric pure (+)-enantiomer.

The compounds of formula I may be prepared by reacting a compound of formula II 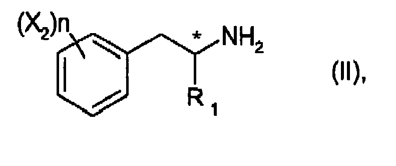 in which R₁, X₂ and n are as defined under formula I and wherein said compound of formula II has an optical activity [α]_{D} of higher than 0° when dissolved in an achiral solvent; with a compound of formula IIIA 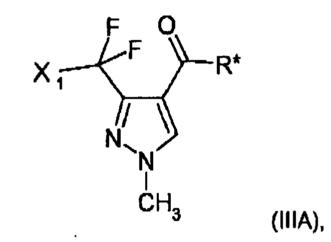 in which X₁ is as defined under formula I, and R* is halogen, hydroxy or C₁₋₆ alkoxy, preferably chloro, in the presence of a base, such as triethylamine, Hunig base, sodium bicarbonate, sodium carbonate, potassium carbonate, pyridine or quinoline, but preferably triethylamine, and in a solvent, such as diethylether, TBME, THF, dichloromethane, chloroform, DMF or NMP, for between 10 minutes and 48 hours, preferably 12 to 24 hours, and between 0° C and reflux, preferably 20 to 25 °C.

When R* is hydroxy, a coupling agent, such as benzotriazol-1-yloxytris(dimethylamino) phosphoniumhexafluorophosphate, bis-(2-oxo-3-oxazolidinyl)-phosphinic acid chloride (BOP-Cl), N,N'-dicyclohexylcarbodiimide (DCC) or 1,1'-carbonyl-dilmidazole (CDI), may be used.

Intermediates of the formula II 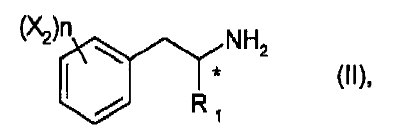 in which R₁, X₂ and n are as defined under formula 1 and wherein said compound of formula II has an optical activity [α]_{D} of higher than 0° when dissolved in an achiral solvent, are novel and were developed specifically for the preparation of compounds of formula I. Accordingly, these intermediates also form part of the subject-matter of the invention.

Intermediates of the formula II may be prepared by the resolution of the according racemic intermediates of formula IIA 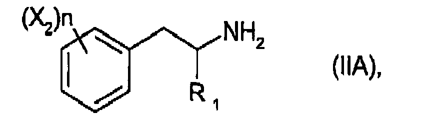 in which R₁, X₂ and in are as defined under formula I and wherein said compound of formula IIA has an optical activity [α]_{D} of 0° when dissolved in an achiral solvent, by chromatography using a suited chiral stationary phase.

The compounds of formula I may also be prepared by reacting a racemic compound of formula IIA 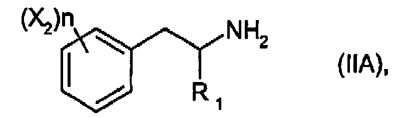 in which R₁, X₂ and n are as defined under formula I and wherein said compound of formula IIA has an optical activity [α]_{D} of 0° when dissolved in an achiral solvent, with a compound of formula IIIA in order to form a racemic compound of formula I, followed by resolution of this racemic compound of formula I by chromatography using a suited chiral stationary phase. The reaction conditions for the amidation are as described above for the reaction of the optically active compound of formula II with the compound of formula IIIA. An example of a suited chiral stationary phase is given in Example P1b).

Racemic intermediates of the formula IIA may be prepared according to the following reaction scheme 1 or in analogy to this reaction scheme.

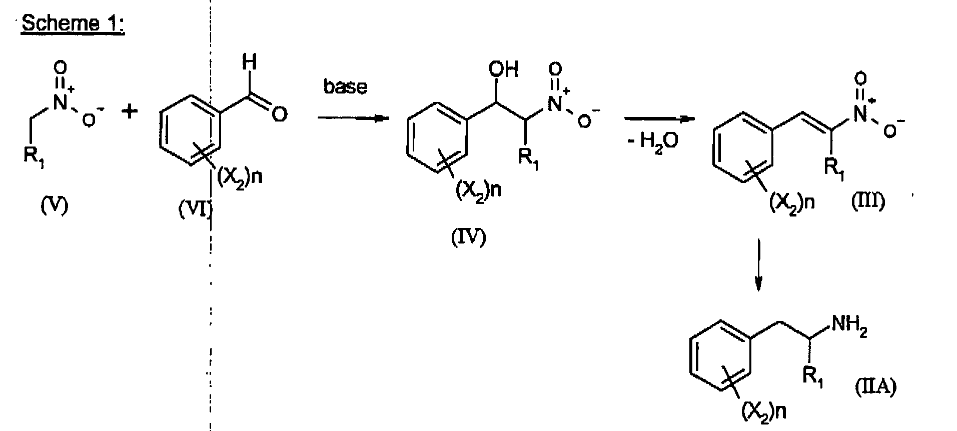

Nitroalkenes of formula III, in which X₂, n and R₁ are as defined under formula IIA, can be prepared by a Henry-reaction (nitroaldol-reaction) of a nitroalkane of formula V, in which R₁ is as defined under formula IIA, with a carbonyl compound of formula (VI), in which X₂ and n are as defined under formula IIA, according to (a) Baer, H. H., Urbas, L. The chemistry of the nitro and nitroso groups; Feuer, H., Ed.; Interscience: New York, 1970; Vol.2,pp 75-20 ; (b) Schickh, G.; Apel, H. G. Methoden der Organischen Chemie (Houben-Weyl Stuttgart, 1971; Vol. 10/1, pp 9-462*;* (c) Kabalka, G. W.; Varma, R.s. Org. Prep. Proc. Int. 1987, 283-328; or (d) Luzzio, F. A. Tetrahedron 2001, 57, 915-945; followed by a dehydration step of the resultant 2-nitro alcohol intermediates of formula IV, in which which X₂, n and R₁ are as defined under formula IIA. Such a dehydration step is described, for example, in Org. Synthesis Coll Vol I, 413, (1941***).*** The mentioned reactions are carried out at temperatures of between 0 - 80°C in convenient protic and aprotic solvents, but also under solvent-free conditions. Convienient bases described in the literature include alkali metal hydroxides, alkaline earth oxides, carbonates, bicarbonates, alkoxides and quarternary ammonium salts. Reduction of the nitroalkenes III may be accomplished using lithium aluminium hydride in an ether solvent such as diethyl ether, ethylene glycol dimethyl ether, diethylene glycol dimethyl ether, tetrahydrofuran or dioxane, or by catalytic reduction over Raney nickel or a noble metal catalyst. The reduction is carried out at temperatures of between 20 - 80°C.

Intermediates of the formula III may also be prepared according to the following reaction scheme 2.

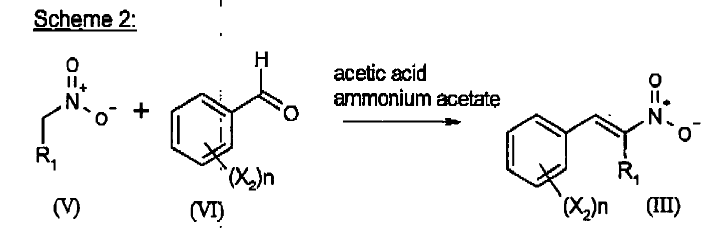

Nitroalkenes of formula III, in which X₂, n and R₁ are as defined under formula IIA, can be prepared by the reaction of a nitroalkane of formula V, in which R₁ is as defined under formula IIA, with a carbonyl compound of formula (VI), in which X₂ and n are as defined under formula IIA, in the presence of acetic acid and ammonium acetate at temperatures between ambient temperature and reflux temperature.

For preparing all further compounds of the formula I functionalized according to the definitions of X₁, R₁, X₂ and n there are a large number of suitable known standard methods, such as alkylation, halogenation, acylation, amidation, oximation, oxidation and reduction. The choice of the preparation methods which are suitable are depending on the properties (reactivity) of the substituents in the intermediates.

The compounds of the formula IIIA are known and some of them are commercially available. They can be prepared analogously as described, for example, in WO 93/11117. The compounds of formula V and VI are known and are commercially available or can be prepared according to the above-mentioned references or according to methods known in the art.

The reactions leading to compounds of the formula I are advantageously carried out in aprotic inert organic solvents. Such solvents are hydrocarbons such as benzene, toluene, xylene or cyclohexane, chlorinated hydrocarbons such as dichloromethane, trichloromethane, tetrachloromethane or chlorobenzene, ethers such as diethyl ether, ethylene glycol dimethyl ether, diethylene glycol dimethyl ether, tetrahydrofuran or dioxane, nitriles such as acetonitrile or propionitrile, amides such as N,N-dimethylformamide, diethylformamide or N-methylpyrrolidinone. The reaction temperatures are advantageously between -20°C and +120°C. In general, the reactions are slightly exothermic and, as a rule, they can be carried out at room temperature. To shorten the reaction time, or else to start the reaction, the mixture may be heated briefly to the boiling point of the reaction mixture. The reaction times can also be shortened by adding a few drops of base as reaction catalyst. Suitable bases are, in particular, tertiary amines such as trimethylamine, triethylamine, quinuclidine, 1,4-diazabicyclo[2.2.2]octane, 1,5-diazabloyclo[4.3.0]non-5-ene or 1,5-diazabicyclo[5.4.0]undec-7-ene. However, inorganic bases such as hydrides, e.g. sodium hydride or calcium hydride, hydroxides, e.g. sodium hydroxide or potassium hydroxide, carbonates such as sodium carbonate and potassium carbonate, or hydrogen carbonates such as potassium hydrogen carbonate and sodium hydrogen carbonate may also be used as bases. The bases can be used as such or else with catalytic amounts of a phase-transfer catalyst, for example a crown ether, in particular 18-crown-6, or a tetraalkylammonium salt.

The compounds of formula I can be isolated in the customary manner by concentrating and/or by evaporating the solvent and purified by recrystallization or trituration of the solid residue in solvents in which they are not readily soluble, such as ethers, aromatic hydrocarbons or chlorinated hydrocarbons. The compounds of formula I can, if appropriate, also be obtained in the form of hydrates and/or include other solvents, for example those which may have been used for the crystallization of compounds which are present in solid form.

It has now been found that the compounds of formula I have, for practical purposes, a very advantageous spectrum of activities for protecting useful plants against diseases that are caused by phytopathogenic microorganisams, such as fungi, bacteria or viruses.

The invention relates to a method of controlling or preventing infestation of useful plants by phytopathogenic microorganisms, wherein a compound of formula I is applied as acitve ingredient to the plants, to parts thereof or the locus thereof. The compounds of formula I according to the invention are distinguished by excellent activity at low rates of application, by being well tolerated by plants and by being environmentally safe. They have very useful curative, preventive and systemic properties and are used for protecting numerous useful plants. The compounds of formula I can be used to inhibit or destroy the diseases that occur on plants or parts of plants (fruit, blossoms, leaves, stems, tubers, roots) of different crops of useful plants, while at the same time protecting also those parts of the plants that grow later e.g. from phytopathogenic microorganisms.

It is also possible to use compounds of formula I as dressing agents for the treatment of plant propagation material, in particular of seeds (fruit, tubers, grains) and plant cuttings (e.g. rice), for the protection against fungal infections as well as against phytopathogenic fungi occurring in the soil.

Furthermore the compounds of formula I according to the invention may be used for controlling fungi in related areas, for example in the protection of technical materials, including wood and wood related technical products, in food storage or in hygiene management.

The compounds of formula I are, for example, effective against the phytopathogenic fungi of the following classes: Fungi imperfecti (e.g. Botrytis, Pyricularia, Helminthosporium, Fusarium, Septoria, Cercospora and Alternaria) and Basidiomycetes (e.g. Rhizoctonia, Hemileia, Puccinia). Additionally, they are also effective against the Ascomycetes classes (e.g. Venturia and Erysiphe, Podosphaera, Monilinia, Uncinula) and of the Oomycetes classes (e.g. Phytophthora, Pythium, Plasmopara). Outstanding activity has been observed against powdery mildew (Erysiphe spp.). Furthermore, the novel compounds of formula I are effective against phytopathogenic bacteria and viruses (e.g. against Xanthomonas spp, Pseudomonas spp, Erwinia amylovora as well as against the tobacco mosaic virus). Good activity has been observed against Asian soybean rust (Phakopsora pachyrhizi).

Within the scope of the invention, useful plants to be protected typically comprise the following species of plants: cereal (wheat, barley, rye, oat, rice, maize, sorghum and related species); beet (sugar beet and fodder beet); pomes, drupes and soft fruit (apples, pears, plums, peaches, almonds, cherries, strawberries, raspberries and blackberries); leguminous plants (beans, lentils, peas, soybeans); oil plants (rape, mustard, poppy, olives, sunflowers, coconut, castor oil plants, cocoa beans, groundnuts); cucumber plants (pumpkins, cucumbers, melons); fibre plants (cotton, flax, hemp, jute); citrus fruit (oranges, lemons, grapefruit, mandarins); vegetables (spinach, lettuce, asparagus, cabbages, carrots, onions, tomatoes, potatoes, paprika); lauraceae (avocado, cinnamomum, camphor) or plants such as tobacco, nuts, coffee, eggplants, sugar cane, tea, pepper, vines, hops, bananas and natural rubber plants, as well as ornamentals.

The term "useful plants" is to be understood as including also useful plants that have been rendered tolerant to herbicides like bromoxynil or classes of herbicides (such as, for example, HPPD inhibitors, ALS inhibitors, for example primisulfuron, prosulfuron and trifloxysulfuron, EPSPS (5-enol-pyrovyl-shikimate-3-phosphate-synthase) inhibitors, GS (glutamine synthetase) inhibitors or PPO (protoporphyrinogen-oxidase) inhibitors) as a result of conventional methods of breeding or genetic engineering. An example of a crop that has been rendered tolerant to imidazolinones, e.g. imazamox, by conventional methods of breeding (mutagenesis) is ClearField® summer rape (Canola). Examples of crops that have been rendered tolerant to herbicides or classes of herbicides by genetic engineering methods include glyphosate- and glufosinate-resistant maize varieties commercially available under the trade names RoundupReady®, Herculex I® and LibertyLink®.

The term "useful plants" is to be understood as including also useful plants which have been so transformed by the use of recombinant DNA techniques that they are capable of synthesising one or more selectively acting toxins, such as are known, for example, from toxin-producing bacteria, especially those of the genus Bacillus.

The term "useful plants" is to be understood as including also useful plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO 95/33818, and EP-A-0 353 191. The methods of producing such transgenic plants are generally known to the person skilled in the art and are described, for example, in the publications mentioned above.

The term locus" of a useful plant as used herein is intended to embrace the place on which the useful plants are growing, where the plant propagation materials of the useful plants are sown or where the plant propagation materials of the useful plants will be placed into the soil. An example for such a locus is a field, on which crop plants are growing.

The term "plant propagation material" is understood to denote generative parts of the plant, such as seeds, which can be used for the multiplication of the latter, and vegetative material, such as cuttings or tubers, for example potatoes. There may be mentioned for example seeds (in the strict sense), roots, fruits, tubers, bulbs, rhizomes and parts of plants. Germinated plants and young plants which are to be transplanted after germination or after emergence from the soil, may also be mentioned. These young plants may be protected before transplantation by a total or partial treatment by immersion. Preferably "plant propagation material" is understood to denote seeds.

The compounds of formula I can be used in unmodified form or, preferably, together with carriers and adjuvants conventionally employed in the art of formulation.

Therefore the invention also relates to compositions for controlling and protecting against phytopathogenic microorganisms, comprising a compound of formula I and an inert carrier, and to a method of controlling or preventing infestation of useful plants by phytopathogenic microorganisms, wherein a composition, comprising a compound of formula I as acitve ingredient and an inert carrier, is applied to the plants, to parts thereof or the locus thereof.

To this end compounds of formula I and inert carriers are conveniently formulated in known manner to emulsifiable concentrates, coatable pastes, directly sprayable or dilutable solutions, dilute emulsions, wettable powders, soluble powders, dusts, granulates, and also encapsulations e.g. in polymeric substances. As with the type of the compositions, the methods of application, such as spraying, atomising, dusting, scattering, coating or pouring, are chosen in accordance with the intended objectives and the prevailing circumstances.
The compositions may also contain further adjuvants such as stabilizers, antifoams, viscosity regulators, binders or tackifiers as well as fertilizers, micronutrient donors or other formulations for obtaining special effects.

Suitable carriers and adjuvants can be solid or liquid and are substances useful in formulation technology, e.g. natural or regenerated mineral substances, solvents, dispersants, wetting agents, tackifiers, thickeners, binders or fertilizers. Such carriers are for example described in WO 97/33890.

The compounds of formula or compositions, comprising a compound of formula I as acitve ingredient and an inert carrier, can be applied to the focus of the plant or plant to be treated, simultaneously or in succession with further compounds. These further compounds can be e.g. fertilizers or micronutrient donors or other preparations which influence the growth of plants. They can also be selective herbicides as well as insecticides, fungicides, bactericides, nematicides, molluscicides or mixtures of several of these preparations, if desired together with further carriers, surfactants or application promoting adjuvants customarily employed in the art of formulation.

A preferred method of applying a compound of formula 1, or a composition, comprising a compound of formula I as acitve ingredient and an inert carrier, is foliar application. The frequency of application and the rate of application will depend on the risk of infestation by the corresponding pathogen. However, the compounds of formula I can also penetrate the plant through the roots via the soil (systemic action) by drenching the locus of the plant with a liquid formulation or by applying the compounds in solid form to the soil, e.g. in granular form (soil application). In crops of water rice such granulates can be applied to the flooded rice field. The compounds of formula I may also be applied to seeds (coating) by impregnating the seeds or tubers either with a liquid formulation of the fungicide or coating them with a solid formulation.

A formulation, i.e. a composition comprising the compound of formula I and, if desired, a solid or liquid adjuvant, is prepared in a known manner, typically by intimately mixing and/or grinding the compound with extenders, for example solvents, solid carriers and, optionally, surface-active compounds (surfactants).

The agrochemical formulations will usually contain from 0.1 to 99% by weight, preferably from 0.1 to 95% by weight, of the compound of formula 1, 99.9 to 1% by weight, preferably 99.8 to 5% by weight, of a solid or liquid adjuvant, and from 0 to 25% by weight, preferably from 0.1 to 25% by weight, of a surfactant.

Whereas it is preferred to formulate commercial products as concentrates, the end user will normally use dilute formulations.

Advantageous rates of application are normally from 5g to 2kg of active ingredient (a.i.) per hectare (ha), preferably from 10g to 1 kg a.i./ha. most preferably from 20g to 600g a.i./ha. When used as seed drenching agent, convenient rates of application are from 10mg to 1g of active substance per kg of seeds. The rate of application for the desired action can be determined by experiments. It depends for example on the type of action, the developmental stage of the useful plant, and on the the application (location, timing, application method) and can, owing to these parameters, vary within wide limits.

Surprisingly, it has now been found that the compounds of formula I can also be used in methods of protecting crops of useful plants against attack by phytopathogenic organisms as well as the treatment of crops of useful plants infested by phytopathogenic organisms comprising administering a combination of glyphosate and at least one compound of formula I to the plant or locus thereof, wherein the plant is resistant or sensitive to glyphosate.

Said methods may provide unexpectedly improved control of diseases compared to using the compounds of formula I in the absence of glyphosate. Said methods may be effective at enhancing the control of disease by compounds of formula I. While the mixture of glyphosate and at least one compound of formula I may increase the disease spectrum controlled, at least in part, by the compound of formula 1, an increase in the activity of the compound of formula I on disease species already known to be controlled to some degree by the compound of formula I can also be the effect observed.

Said methods are particularly effective against the phytopathogenic organisms of the kingdom *Fungi,* phylum *Basidiomycot,* class *Uredinomycetes,* subclass *Urediniomycetidae* and the order *Uredinales* (commonly referred to as rusts). Species of rusts having a particularly large impact on agriculture include those of the family Phakopsoraceae, particularly those of the genus *Phakopsora,* for example *Phakopsora pachyrhizi,* which is also referred to as Asian soybean rust, and those of the family *Pucciniaceae,* particularly those of the genus *Puccinia* such as *Puccinia graminis,* also known as stem rust or black rust, which is a problem disease in cereal crops and *Puccinia recondita*, also known as brown rust.

An embodiment of said method is a method of protecting crops of useful plants against attack by a phytopathogenic organism and/or the treatment of crops of useful plants infested by a phytopathogenic organism, said method comprising simultaneously applying glyphosate, including salts or esters thereof, and at least one compound of formula I, which has activity against the phytopathogenic organism to at least one member selected from the group consisting of the plant, a part of the plant and the locus of the plant.

Surprisingly, it has now been found that the compounds of formula I, or a pharmaceutical salt thereof, described above have also an advantageous spectrum of activity for the treatment and/or prevention of microbial infection in an animal.

"Animal" can be any animal, for example, insect, mammal, reptile, fish, amphibian, preferably mammal, most preferably human. "Treatment" means the use on an animal which has microbial infection in order to reduce or slow or stop the increase or spread of the infection, or to reduce the infection or to cure the infection. "Prevention" means the use on an animal which has no apparent signs of microbial infection in order to prevent any future infection, or to reduce or slow the increase or spread of any future infection.

According to the present invention there is provided the use of a compound of formula I in the manufacture of a medicament for use in the treatment and/or prevention of microbial infection in an animal. There is also provided the use of a compound of formula I as a pharmaceutical agent. There is also provided the use of a compound of formula I as an antimicrobial agent in the treatment of an animal. According to the present invention there is also provided a pharmaceutical composition comprising as an active ingredient a compound of formula I, or a pharmaceutically acceptable salt thereof, and a pharmaceutically acceptable diluent or carrier. This composition can be used for the treatment and/or prevention of antimicrobial infection in an animal. This pharmaceutical composition can be in a form suitable for oral administration, such as tablet, lozenges, hard capsules, aqueous suspensions, oily suspensions, emulsions dispersible powders, dispersible granules, syrups and elixirs. Alternatively this pharmaceutical composition can be in a form suitable for topical application, such as a spray, a cream or lotion. Alternatively this pharmaceutical composition can be in a form suitable for parenteral administration, for example injection. Alternatively this pharmaceutical composition can be in inhalable form, such as an aerosol spray.

The compounds of formula I are effective against various microbial species able to cause a microbial infection in an animal. Examples of such microbial species are those causing Aspergillosis such as *Aspergillus fumigatus, A. flavus, A. terrus,* A. *nidulans* and *A. niger,* those causing Blastomycosis such as *Blastomyces dermatitidis;* those causing Candidiasis such as *Candida albicans,* C. *glabrata, C. tropicalis,* C. *parepsilosis, C. krusei* and C. *lusitaniae;* those causing Coccidioidomycosis such as Coccidioides *immitis;* those causing Cryptococcosis such as *Cryptococcus neoformans;* those causing Histoplasmosis such as *Histoplasma capsuletum* and those causing Zygomycosis such as *Absidia corymbifera, Rhizomucor pusillus* and *Rhizopus arrhizus.* Further examples are Fusarium Spp such as *Fusarium oxysporum* and *Fusarium solani* and Scedosporium Spp such as *Scedosporium apiospermum* and *Scedosporium prolificans.* Still further examples are Microsporum Spp, Trichophyton Spp, Epidermophyton Spp, Mucor Spp, Sporothorix Spp, Phialophora Spp, Cladosporium Spp, Petriellidium spp, Paracoccidioides Spp and Histoplasma Spp.

The following non-limiting Examples illustrate the above-described invention in greater detail without limiting it.

### Preparation examples:

### Example P1: Preparation of (+)-3-difluoromethyl-1-methyl-1H-pyrazole-4-carboxyllc acid [2-(2,4-dichloiophenyl)-1-methyl-ethyl]-amide (compound no. 1.01):

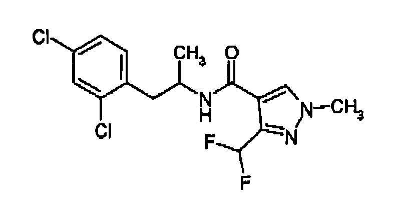

### a) Preparation of racemic 3-difluoromethyl-1-methyl-1H-pvrazole-4-carboxylic acid [2-(2,4-dichlorophenyl)-1-methyl-ethyl]-amide

A solution of 3-difluoromethyl-1-methyl-1 H-pyrazole4-carbonyl chloride (1.95g; 10 mmol) in dichloromethane (10ml) was added dropwise to a stirred solution of 2.04g (10 mmol) 2-(2,4-dichloro-phenyl)-1-methyl-ethylamine, which was prepared as described in example P2, and triethylamine (0.152g; 15mmol) in dichloromethane (30ml). The reaction mixture was stirred for 1 hr at ambient temperature then allowed to stand for 3h. The reaction mixture was washed with 1 M NaOH (20ml) and with 1 M HCl (20ml) and then dried over Na₂SO₄. After removal of the solvent the residue was purified by flash chromatography over silica gel (eluant: hexane/ethyl acetate 1:1). 2.21g (61% of theory) of 3-diftuoromethyf-1-methyl-1H-pyrazole-4-carboxylic acid [2-(2,4-dichlorophenyl)-1-methyl-ethyl]-amide was obtained in the form of a solid (m.p.157°C).
¹H NMR (400MHz, CDCl₃): δ 1.24(d,3H,C*H*₃), 2.95(m,2H,C*H*₂), 3.90(s,3H,NC*H*₃), 4.46(m,1H,CH), 6.21(t,1H,NH), 6.80(t,1H,C*H*F₂), 7.14-7.19(m,2H,Ar-H), 7.37(d,1H,Ar-H), 7.84(s,1H,pyrazole-H).
MS [M+H]⁺ 362/364/366.

### b) Preparation of (+)-3-difluoromethyl-1-methyl-1H-pyrazole-4-carboxylic acid [2-(2,4-dichlorophenyl)-1-methyl-ethyl]-amide (compound 1.01)

Racemic 3-difluorormethyl-1-methyl-1H-pyrazole-4-carboxylic acid [2-{2,4-dichlorophenyl)-1-methyl-ethyn-amidel (480mg, prepared as described in Example 1a) was dissolved in n-hexanerisopropanoll 3:1 (v/v) 72ml. The solution was purified on Chiralpak AD® (Lot No. AD00CM-BF001 Daicel Japan, dimension: 500mm x 50mm, particle size: 20 µm, flow rate: 30ml/min) using n-hexane/ isopropanol 7:3 (v/v) as eluant on high performance liquid chromatography (HPLC). For the separation of the whole material runs of 8ml each (53mg of the racemate) were separated on the column. The detection of the compounds was performed with UV detector at 210nm. Pure enantiomeric samples (ee > 99%) checked by analytical HPLC (Chiralpak AD00CE-CH017, Daicel) were combined and the solvent was evaporated.

Optical rotation data has been collected on a Perkin Elmer 241 Polarimeter (compounds were dissolved in CHCl₃, temperature is given in degrees Celsius; "c" stands for concentration in g/ml, the optical path length was 10cm).

### Compound 1.01. (+)-compound:

116mg of (+)-3-difluoromethyl-1-methyl-1H-pyrazole-4-carboxylic acid [2-(2,4-dichlorophenyl)-1-methyl-ethyl]-amide was obtained in the form of a solid (Chiralpak AD00CE-CH017, Daicel, n-hexanerisopropanol 85:15; Retention time; 10.34 min); [α]²³_{D}=+ 50 (c 4.9. CHCl₃).

### Comparative Example: Compound 1.01, (-)-compound:

174mg of (-)-3-diflupromethyl-1-methyl-1H-pyrazote-4-carboxylic acid [2-(2,4-dichlorophenyl)-1-methyl-ethyl]-amide was obtained in the form of a solid (Chiralpak ADOOCE-CH017, Daicel, n-hexane/isopropanol 85:15; Retention time: 7.80 min); [α]²³_{D}=-52 (c 4.4,CHCl₃).

### Example P2: Preparation of 2-(2,4-dichlorophenyl)-1-methyl-ethylamine:

### a) Preparation of 2,4-dichloro-1-((E)-2-nitro-propenyl)-benzene

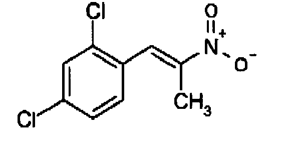

In a sulfonation flask 2,4-dichloro-benzeldehyde (77g, 0.44mol), nitroethane (216ml, 3.04mol) and ammonium acetate (81.4g, 1.06mol) were added to glacial acetic acid (600ml). The resulting solution was heated to 90°C for three hours. After removal of the solvent ice-water (400ml) was added. The solid product was collected by filtration, washed with water and recrystallized from ethanol. 55.9 g (55% of theory) of 2,4-dichloro-1-((E)-2-nitro-propenyl)-benzene was obtained in the form of a yellow solid (m.p. 79-81 °C).
¹H NMR (400MHz, CDCl₃): δ 8.11 (s,1H), 7.51 (d, 1H), 7.34 (dd, 1H), 7.27(d,1 H), 2.33(s,3H,CH₃).

### b) Preparation of 2-(2,4-dichlorophenyl)-1-methyl-ethylamine

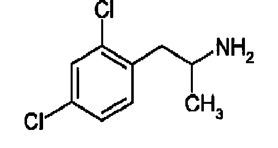

To a stirred suspension of lithium aluminium hydride (3 equiv, 30 mmol. 1.14 g) in dry tetrahydrofurane (30 ml) under nitrogen atmosphere was added dropwise a solution of 2,4-dichloro-1-((E)-2-nitro-propenyl)-benzene (10 mmol, 2.32 g) in dry THF (20ml) under cooling with an ice bath. After stirring for 10 minutes the suspension was heated to reflux for 1 hour, then the mixture was cooled to 0°C and excess lithium aluminium hydride was decomposed by the sequential dropwise addition of water (40ml), tert.-butylmethylether (20ml), 20% NaOH (20ml) and water (40ml) under stirring. The reaction product was collected by filtration and washed with MTBE. The filtrate was washed with brine, dried over MgSO₄. filtered and dried under reduced pressure. 2.0g (98% of theory) of 2-(2,4-dichlorophenyl)-1-methyl-ethylamine (compound Z1.01) was obtained in the form of a brown oil.
MS [M+H]⁺ 204/206/208.
The 2-(2,4-dichlorophenyl)-1-methyl-ethylamine was used in example P1 without further purification.

### Table 1: Compounds of formula IA

The invention is further illustrated by the prefered individual compounds of formula (IA) listed below in Table 1.

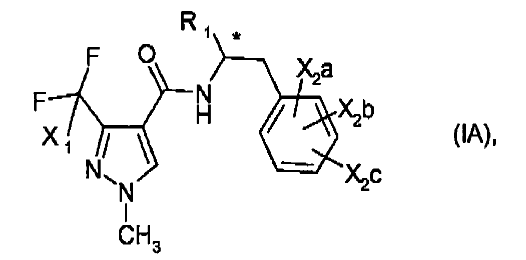

The [α]²³_{D} value is measured by dissolving the compound of formula I in chloroform and measuring its optical activity at λ=589 nm and 23°C with an optical path length of 10cm. "C" stands for concentration and is measured in g/ml.

**Table 1:**

| **Comp. No.** | **X₁** | **R₁** | **X₂ₐ** | **X_{2b}** | **X_{2c}** | **[α]²³_{D}** |
|---|---|---|---|---|---|---|
| 1.01 | H | CH₃ | 2-Cl | 4-Cl | H | + 50 c=4.9 |
| 1.02 | H | CH₃ | 2-Cl | 6-Cl | 4-Cl | - |
| 1.03 | H | CH₃ | 2-Cl | 6-Cl | H | - |
| 1.04 | H | CH₃ | 2-Cl | 6-CH₃ | H | - |
| 1.05 | H | CH₃ | 2-Cl | 6-CF₃ | H | - |
| 1.06 | H | CH₃ | 2-Cl | 6-Cl | 4-CF₃ | - |
| 1.07 | H | CH₃ | 2-Cl | 6-Cl | 4-CH₃ | - |
| 1.08 | H | CH₃ | 2-Cl | 6-Cl | 4-Br | - |
| 1.09 | F | CH₃ | 2-Cl | 4-Cl | H | - |
| 1.10 | F | CH₃ | 2-Cl | 6-Cl | 4-Cl | - |
| 1.11 | F | CH₃ | 2-Cl | 6-Cl | H | - |
| 1.12 | F | CH₃ | 2-Cl | 6-CH₃ | H | - |
| 1.13 | F | CH₃ | 2-Cl | 6-CF₃ | H | - |
| 1.14 | F | CH₃ | 2-Cl | 6-Cl | 4-CF₃ | - |
| 1.15 | F | CH₃ | 2-Cl | 6-Cl | 4-CH₃ | - |
| 1.16 | F | CH₃ | 2-Cl | 6-Cl | 4-Br | - |
| 1.17 | H | C₂H₅ | 2-Cl | 4-Cl | H | - |
| 1.18 | H | C₂H₅ | 2-Cl | 6-Cl | 4-Cl | - |
| 1.19 | H | C₂H₅ | 2-Cl | 6-Cl | H | - |
| 1.20 | H | C₂H₅ | 2-Cl | 6-CH₃ | H | - |
| 1.21 | H | C₂H₅ | 2-Cl | 6-CF₃ | H | - |
| 1.22 | H | C₂H₅ | 2-Cl | 6-Cl | 4-CF₃ | - |
| 1.23 | H | C₂H₅ | 2-Cl | 6-Cl | 4-CH₃ | - |
| 1.24 | H | C₂H₅ | 2-Cl | 6-Cl | 4-Br | - |

### Table 2: Compounds of formula IIA

The invention is further illustrated by the prefered individual compounds of formula (IIA) listed below in Table 2.

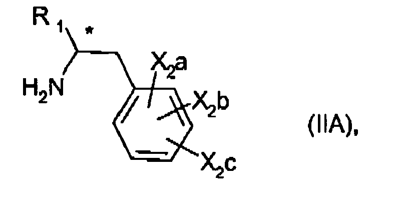

**Table 2:**

| **Comp. No.** | **R₁** | **X₂ₐ** | **X_{2b}** | **X_{2c}** | **[α]²³_{D}** |
|---|---|---|---|---|---|
| Z1.01 | CH₃ | 2-Cl | 4-Cl | H | - |
| Z1.02 | CH₃ | 2-Cl | 6-Cl | 4-Cl | - |
| Z1.03 | CH₃ | 2-Cl | 6-Cl | H | - |
| Z1.04 | CH₃ | 2-Cl | 6-CH₃ | H | - |
| Z1.05 | CH₃ | 2-Cl | 6-CF₃ | H | - |
| Z1.06 | CH₃ | 2-Cl | 6-Cl | 4-CF₃ | - |
| Z1.07 | CH₃ | 2-Cl | 6-Cl | 4-CH₃ | |
| Z1.08 | CH₃ | 2-Cl | 6-Cl | 4-Br | - |
| Z1.09 | CH₃ | 2-Cl | 4-Cl | H | - |
| Z1.10 | CH₃ | 2-Cl | 6-Cl | 4-Cl | - |
| Z1.11 | CH₃ | 2-Cl | 6-Cl | H | - |
| Z1.12 | CH₃ | 2-Cl | 6-CH₃ | H | - |
| Z1.13 | CH₃ | 2-Cl | 6-CF₃ | H | - |
| Z1.14 | CH₃ | 2-Cl | 6-Cl | 4-CF₃ | - |
| Z1.15 | CH₃ | 2-Cl | 6-Cl | 4-CH₃ | - |
| Z1.16 | CH₃ | 2-Cl | 6-Cl | 4-Br | - |
| Z1.17 | C₂H₅ | 2-Cl | 4-Cl | H | - |
| Z1.18 | C₂H₅ | 2-Cl | 6-Cl | 4-Cl | - |
| Z1.19 | C₂H₅ | 2-Cl | 6-Cl | H | - |
| Z1.20 | C₂H₅ | 2-Cl | 6-CH₃ | H | - |
| Z1.21 | C₂H₅ | 2-Cl | 6-CF₃ | H | - |
| Z1.22 | C₂H₅ | 2-Cl | 6-Cl | 4-CF₃ | - |
| Z1.23 | C₂H₅ | 2-Cl | 6-Cl | 4-CH₃ | - |
| Z1.24 | C₂H₅ | 2-Cl | 6-Cl | 4-Br | - |

### FORMULATION EXAMPLES FOR COMPOUNDS OF FORMULA I:

### Example F-1.1 to F-1.3: Emulsifiable concentrates.

| Components | F-1.1 | F-1.2 | F-1.3 |
|---|---|---|---|
| compound of Table 1 | 25% | 40% | 50% |
| calcium dodecylbenzenesulfonate | 5% | 8% | 6% |
| castor oil polyethylene glycol ether (36 mol ethylenoxy units) | 5% | - | - |
| tributylphenolpolyethylene glycol ether (30 mol ethylenoxy units) | - | 12% | 4% |
| cyclohexanone | - | 15% | 20% |
| xylene mixture | 65% | 25% | 20% |

Emulsions of any desired concentration can be prepared by diluting such concentrates with water.

### Example F-2: Emulsifiable concentrate

| Components | F-2 |
|---|---|
| compound of Table 1 | 10% |
| octylphenolpolyethylene glycol ether (4 to 5 mol ethylenoxy units) | 3% |
| calcium dodecylbenzenesulfonate | 3% |
| castor oil polyglycol ether (36 mol ethylenoxy units) | 4% |
| cyclohexanone | 30% |
| xylene mixture | 50% |

Emulsions of any desired concentration can be prepared by diluting such concentrates with water.

### Examples F-3.1 to F-3.4: Solutions

| Components | F-3.1 | | F-3.2 | | F-3.3 | | F-3.4 | |
|---|---|---|---|---|---|---|---|---|
| compound of Table 1 | 80% | | 10% | | 5% | | 95% | |
| propylene glycol monomethyl ether | | 20% | | - | | - | | - |
| polyethylene glycol (relative molecular mass: 400 atomic mass units) | - | | 70% | | - | | - | |
| N-methylpyrrolid-2-one | - | | 20% | | - | | - | |
| epoxidised coconut oil | - | | - | | 1% | | 5% | |
| benzin (boiling rangle: 160-190°) | - | | - | | 94% | | - | |

The solutions are suitable for use in the form of microdrops.

### Examples F-4.1 to F-4.4: Granulates

| Components | F-4.1 | F-4.2 | F-4.3 | F-4.4 |
|---|---|---|---|---|
| compound of Table 1 | 5% | 10% | 8% | 21% |
| kaolin | 94% | - | 79% | 54% |
| highly dispersed silicic acid | 1% | - | 13% | 7% |
| attapulgite | - | 90% | - | 18% |

The novel compound is dissolved in dichloromethane, the solution is sprayed onto the carrier and the solvent is then removed by distillation under vacuum.

### Examples F-5.1 and F-5.2: Dusts

| Components | F-5.1 | F-5.2 |
|---|---|---|
| compound of Table 1 | 2% | 5% |
| highly dispersed silicic acid | 1% | 5% |
| talcum | 97% | - |
| kaolin | - | 90% |

Ready for use dusts are obtained by intimately mixing all components.

### Examples F-6.1 to F-6.3: Wettable powders

| Components | F-6.1 | F-6.2 | F-6.3 |
|---|---|---|---|
| compound of Table 1 | 25% | 50% | 75% |
| sodium lignin sulfonate | 5% | 5% | - |
| sodium lauryl sulfate | 3% | - | 5% |
| sodium diisobutyinaphthalene sulfonate | - | 6% | 10% |
| octylphenolpolyethylene glycol ether (7 to 8 mol ethylenoxy units) | - | 2% | - |
| highly dispersed silicic acid | 5% | 10% | 10% |
| kaolin | 62% | 27% | - |

All components are mixed and the mixture is thoroughly ground in a suitable mill to give wettable powders Which can be diluted with water to suspensions of any desired concentration.

### Example F7: Flowable concentrate for seed treatment

| | |
|---|---|
| compound of Table 1 | 40 % |
| propylene glycol | 5% |
| copolymer butanol PO/EO | 2 % |
| tristyrenephenole with 10-20 moles EO | 2 % |
| 1,2-benzisothiazolin-3-one (in the form of a 20% solution in water) | 0.5 % |
| monoazo-pigment calcium salt | 5% |
| Silicone oil (in the form of a 75 % emulsion in water) | 0.2 % |
| Water | 45.3 % |

The finely ground active ingredient is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### BIOLOGICAL EXAMPLES: FUNGICIDAL ACTIONS

### Example B-1: Action against Botrytis cinerea - fungal growth assay

Conidia of the fungus from cryogenic storage were directly mixed into nutrient broth (PDB potato dextrose broth). After placing a (DMSO) solution of the test compounds into a microtiter plate (96-well format) the nutrient broth containing the fungal spores was added. The test plates were incubated at 24°C and the inhibition of growth was determined photometrically after 48-72hrs. The activity of a compound is expressed as fungal growth inhibition (0 = no growth inhibition, ratings of 80 % to 99 % mean good to very good inhibition, 100 % = complete inhibition).

| Compound | 20 ppm | 6 ppm | 2 ppm |
|---|---|---|---|
| (+)-3-difluoromethyl-1-methyl-1H-pyrazole-4-carboxylic acid [2-(2,4-dichlorophenyl)-1-methyl-ethyl]-amide (compound according to the invention) | 100 | 100 | 80 |
| (-)-3-difluoromethyl-1-methyl-1H-pyrazole-4-carboxylic acid [2-(2,4-dichlorophenyl)-1-methyl-ethyl]-amide (comparative example) | 20 | 20 | 0 |

### Example B-2: Action against Mycosphaerella arachidis (early leaf spot of groundnut; Cercospora arachidicola [anamorph])- fungal growth assay

Conidia of the fungus from cryogenic storage were directly mixed into nutrient broth (PDB potato dextrose broth). After placing a (DMSO) solution of the test compounds into a microtiter plate (96-well format) the nutrient broth containing the fungal spores was added. The test plates were incubated at 24°C and the inhibition of growth was measured photometrically after 6-7 days. The activity of a compound is expressed as fungal growth inhibition (0 = no growth inhibition, ratings of 80 % to 99 % mean good to very good inhibition, 100 % = complete inhibition).

| Compound | 20 ppm | 6 ppm | 2 ppm |
|---|---|---|---|
| (+)-3-difluoromethyl-1-methyl-1H-pyrazole-4-carboxylic acid [2-(2,4-dichlorophenyl)-1-methyl-ethyl]-amide (compound according to the invention) | 100 | 100 | 100 |
| (-)-3-difluoromethyl-1-methyl-1H-pyrazole-4-carboxylic acid [2-(2,4-dichlorophenyl)-1-methyl-ethyl]-amide (comparative example) | 80 | 50 | 0 |

### Example B-3: Action against Septoria tritici - fungal growth assay

Conidia of the fungus from cryogenic storage were directly mixed into nutrient broth (PDB potato dextrose broth). After placing a (DMSO) solution of the test compounds into a microtiter plate (96-well format) the nutrient broth containing the fungal spores was added. The test plates were incubated at 24°C and the inhibition of growth was determined photometrically after 72 hrs. The activity of a compound is expressed as fungal growth inhibition (0 = no growth inhibition, ratings of 80 % to 99 % mean good to very good inhibition, 100 % = complete inhibition).

| Compound | 20 ppm | 6 ppm | 2 ppm |
|---|---|---|---|
| (+)-3-difluoromethyl-1-methyl-1H-pyrazole-4-carboxylic acid [2-(2,4-dichlorophenyl)-1-methyl-ethyl]-amide (compound according to the invention) | 100 | 100 | 100 |
| (-)-3-difluoromethyl-1-methyl-1H-Pyrazole-4-carboxylic acid [2-(2,4-dichlcrophenyl)-1-methyl-ethyl]-amide (comparative example) | 100 | 80 | 0 |

### Example B-4: Action against Monographella nivalis (anamorph: Fusarium nivale. Microdochium nivale; Snow mould) - fungal growth assay

After placing a DMSO-solution (2% Dimethylsulfoxid, 0,025 % Tween 20) of the test compounds into a microtiter plate (96-well format) the nutrient broth containing the fungal spores was added. 40.000 conidia/ml of the fungus from cryogenic storage were directly mixed into nutrient broth (PDB potato dextrose broth). The compounds were tested at a variety of application rates; these rates are shown in parts per million (ppm) in the table. The test plates were incubated at 24°C and the inhibition of growth was measured photometrically after 72 hrs (0 = no growth inhibition, ratings of 80 % to 99 % mean good to very good inhibition, 100 % = complete inhibition).

| Compound | 20 ppm | 6 ppm | 2 ppm |
|---|---|---|---|
| (+)-3-difluoromethyl-1-methyl-1H-pyrazole-4-carboxylic acid [2-(2,4-dichlorophenyl)-1-methyl-ethyl]-amide (compound according to the invention) | 100 | 100 | 100 |
| (-)-3-difluoromethyl-1-methyl-1H-pyrazole-4-carboxylic acid [2-(2,4-dichlorophenyl)-1-methyl-ethyl]-amide (comparative example) | 20 | 0 | 0 |

### Example B-5: Action against Pyrenophora teres (net blotch) on barley

Barley leaf segments are placed on agar in multiwell plates (24-well format) and sprayed with test solutions (200ppm, 60pp, and 20ppm of active ingredient). After drying, the leaf disks are inoculated with a spore suspension of the fungus. After appropriate incubation the activity of a compound is assessed 4 days after inoculation as preventive fungicidal activity (in %).

| Compound | 200 ppm | 60 ppm | 20 ppm |
|---|---|---|---|
| (+)-3-difluoromethyl-1-methyl-1H-pyrazole-4-carboxylic acid [2-(2,4-dichlorophenyl)-1-methyl-ethyl]-amide(compound according to the invention) | 100 | 100 | 100 |
| (-)-3-difluoromethyl-1-methyl-1H-pyrazole-4-carboxylic acid [2-(2,4-dichlorophenyl)-1-methyl-ethyl]-amide (comparative example) | 100 | 100 | 80 |

## Claims

1. A compound of the formula I 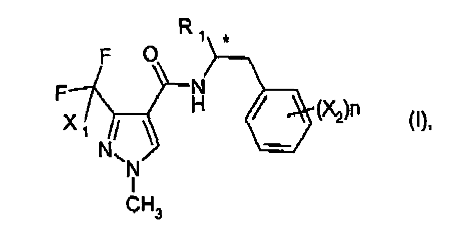 wherein R₁ is C₁-C₃alkyl, CF₃ or CF₂H; X₁ is hydrogen or fluoro; n is 2 or 3; each X₂ independently of each other stands for chloro, bromo, fluoro, CH₃ or CF₃; having an optical activity [α]_{D} of higher than 0° when dissolved in an achiral solvent.

2. A compound of formula according to claim 1, wherein X₁ is hydrogen.

3. A compound of formula I according to claim 1, wherein R₁ is methyl.

4. A compound of formula I according to claim 1, wherein R₁ is ethyl.

5. A compound of formula I according to claim 1, wherein R₁ is CF₃.

6. A compound of formula I according to claim 1, wherein X₂ is chloro.

7. A compound of formula I according to claim 1, wherein n is 2.

8. A compound of formula I according to claim 1, wherein n is 3.

9. A method of controlling or preventing infestation of useful plants by phytopathogenic microorganisms, Wherein a compound of formula I according to claim 1 or a composition, comprising this compound as active ingredient, is applied to the plants, to parts thereof or the locus thereof.

10. A composition for controlling and protecting against phytopathogenic microorganisms, comprising a compound of formula I according to claim 1 and an inert carrier.
